# Europäisches Patentamt

## European Patent Office

## Office européen des brevets

(19)

(11) Veröffentlichungsnummer: **0 093 468**
**B1**

(12) # EUROPÄISCHE PATENTSCHRIFT

(45) Veröffentlichungstag der Patentschrift:
**22.07.87**

(51) Int. Cl.⁴: **H 01 P 1/06, A 61 B 6/00**

(21) Anmeldenummer: **83200560.7**

(22) Anmeldetag: **19.04.83**

(54) Vorrichtung für die Mikrowellenübertragung zwischen zwei relativ zueinander drehbaren Teilen.

(30) Priorität: **24.04.82 DE 3215377**

(43) Veröffentlichungstag der Anmeldung:
**09.11.83 Patentblatt 83/45**

(45) Bekanntmachung des Hinweises auf die Patenterteilung:
**22.07.87 Patentblatt 87/30**

(84) Benannte Vertragsstaaten:
**DE FR GB NL**

(56) Entgegenhaltungen:
**FR-A-2 440 041**
**US-A-2 737 633**
**US-A-2 945 193**
**US-A-3 419 826**

(73) Patentinhaber: **Philips Patentverwaltung GmbH,
Billstrasse 80, D-2000 Hamburg 28 (DE)**

(84) Benannte Vertragsstaaten: **DE**

(73) Patentinhaber: **N.V. Philips' Gloeilampenfabrieken,
Groenewoudseweg 1, NL- 5621 BA Eindhoven (NL)**

(84) Benannte Vertragsstaaten: **FR GB NL**

(72) Erfinder: **Beckmann, Friedrich- Karl, Flagentwiete
44, D-2080 Pinneberg (DE)**
Erfinder: **Hoppe, Wolfgang, Mühlenweg 35, D-2000
Norderstedt (DE)**
Erfinder: **Meyer, Wolfgang, Dr., Eichenweg 16,
D-2359 Henstedt- Ulzburg (DE)**

(74) Vertreter: **Hartmann, Heinrich, Dipl.- Ing., Philips
Patentverwaltung GmbH Billstrasse 80 Postfach
10 51 49, D-2000 Hamburg 28 (DE)**

EP 0 093 468 B1

**Beschreibung**

Die Erfindung betrifft eine Vorrichtung gemäß dem Oberbegriff des Patentanspruches 1.

Eine solche Vorrichtung ist aus der US-PS 3 419 826 bekannt. Als Anwendungsgebiete sind dabei Telefonverbindungen für rotierende Appartementhäuser genannt sowie eine Radarantenne oder ein Flughafenkontrollturm.

Die Notwendigkeit der kontaktlosen Informationsübertragung ist aber auch bei Geräten für die Computertomographie gegeben, wobei von einem Röntgenstrahler emittierte Strahlung einen Patienten durchsetzt und von einer Vielzahl von Strahlendetektoren erfaßt wird, die zusammen mit dem Strahler auf einem um den Patienten rotierenden Teil befestigt sind. Dabei müssen die Detektorsignale von dem rotierenden Teil zu einem feststehenden Teil übertragen werden, damit sie von einem Computer verarbeitet werden können. Wenn der Strahler dabei mehrmals um den Patienten herum rotiert, ist eine Übertragung über Kabel nicht mehr möglich. Die Informationsübertragung kann dabei mittels der eingangs genannten Vorrichtung erfolgen, wenn die Übertragungsgeschwindigkeit bzw. der Datenfluß genügend groß ist.

Bei der bekannten Vorrichtung hat der eine Hohlleiter die Form eines Halbkreises und ist an seinen beiden Enden durch Dämpfungsglieder abgeschlossen, während der andere Hohlleiter zu einem geschlossenen Ring gebildet ist, in dem ein Dämpfungsglied angeordnet ist, das das mehrfache Umlaufen einer Welle in dem Ring verhindern soll. Es ist ein Umschalter vorgesehen, der je nach Position der in die Koppelschlitze hineinragenden Empfangsantennen die eine oder andere Empfangsantenne wirksam macht. Da in dem Hohlleiterring eine elektromagnetische Welle unter Umständen nahezu einen vollständigen Umlauf machen muß, bevor sie die Empfangsantenne erreicht, während in dem halbkreisförmigen Hohlleiter der maximale Weg nur halb so groß ist, ergeben sich bei der Umschaltung der Empfangsantennen Laufzeitsprünge, die eine kontinuierliche Signalübertragung mit hoher Übertragungsrate erheblich erschweren.

Diese Laufzeitsprünge treten bei der Ausführungsform gemäß Fig. 6 der US-PS 3 419 826 zwar nicht auf, doch sind dabei zwei Umschalter erforderlich, die abwechselnd eine von zwei Sendeantennen mit einer von zwei Empfangsantennen verbinden. Die Umschalter müssen jeweils im richtigen Moment umgeschaltet werden, was eine aufwendige Elektronik erfordert.

Aufgabe der vorliegenden Erfindung ist es, eine Vorrichtung der eingangs genannten Art so auszugestalten, daß eine Umschaltung zwischen den Sende- bzw. den Empfangsantennen nicht erforderlich ist.

Erfindungsgemäß wird diese Aufgabe durch die im Kennzeichen des Patentanspruches 1 angegebenen Merkmale gelöst.

Durch die den Hohlleiter abschließende feste Antenne und das Dämpfungsglied wird jeder Hohlleiter in einen aktiven und in einen passiven Bereich unterteilt. Taucht die relativ zum Hohlleiter verschiebbare Antenne im aktiven Bereich in den Koppelschlitz ein, ergibt sich eine Signalübertragung zwischen Sende- und Empfangsantenne. Taucht sie hingegen in den passiven Bereich ein, ist eine Übertragung zwischen den beiden Antennen praktisch ausgeschlossen. Die aktiven und die passiven Bereiche der beiden Hohlleiter sind nun so angeordnet, daß in jeder Position der beiden in den Koppelschlitz eintauchenden Antennen wenigstens eine Antenne in den aktiven Bereich des Hohlleiters eintaucht. Durch die geschilderte Ausgestaltung wird erreicht, daß bei einer Verschiebung der in die Koppelschlitze eintauchenden Antennen auf dem Umfang der Hohlleiter die Laufzeit der elektromagnetischen Welle zwischen der Empfangs- und der Sendeantenne eines Hohlleiters zunächst zunimmt, bis die in den Koppelschlitz eintauchende Antenne den Ort erreicht hat, in dem das Dämpfungsglied befestigt ist. In diesem Augenblick hat auch die andere in den Koppelschlitz eintauchende Antenne den aktiven Bereich erreicht, wobei die Laufzeiten in beiden Hohlleitern gleich sind. Bei einer weiteren Drehung ist die andere in den Koppelschlitz eintauchende Antenne für die Mikrowellenübertragung wirksam (während die erstgenannte in den passiven Bereich gelangt und dort unwirksam wird), wobei die Laufzeit bis auf ein Minimum abnimmt, wonach die eine Antenne wieder den aktiven Bereich erreicht, während die andere Antenne in den passiven Bereich überwechselt. Bei einer kontinuierlichen Drehung ändert sich dabei die Laufzeit also nicht sprunghaft, sondern ebenfalls nur kontinuierlich, so daß hohe Übertragungsgeschwindigkeiten grundsätzlich möglich sind.

Die Erfindung wird nachstehend anhand der Zeichnungen näher erläutert. Es zeigen

Fig. 1 eine Draufsicht in Richtung der Ringachse auf die Hohlleiteranordnung,

Fig. 2 eine perspektivische auseinandergezogene und nicht maßstäbliche Ansicht,

Fig. 3 das Blockschaltbild einer Schaltung zur Speisung der Sendeantennen, und

Fig. 4 das Blockschaltbild einer Schaltung zur Verarbeitung der Signale der Empfangsantennen.

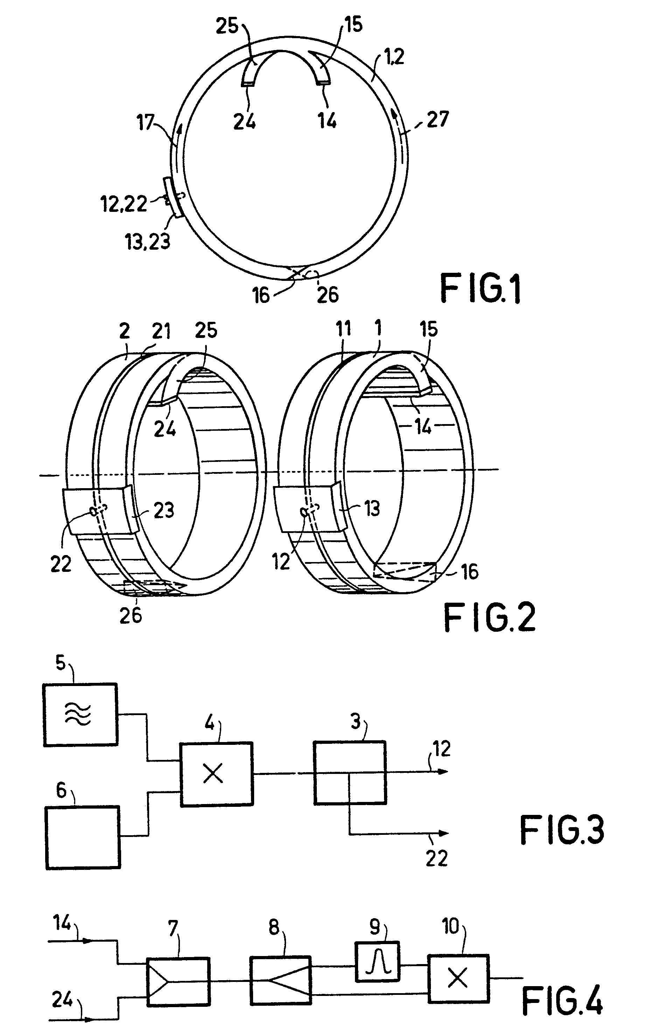

Die Fig. 1 und insbesondere 2 zeigen zwei ringförmig gebogene Hohlleiter 1, 2 mit rechteckigem Querschnitt, die in der Mitte ihres Außenmantels einen Koppelschlitz 11 bzw. 21 aufweisen, in die je eine Sendeantenne 12 bzw. 22 hineinragt. Die Sendeantennen 12 bzw. 22 sind auf je einem Schlitten 13 bzw. 23 befestigt, der auf dem zugeordneten Hohlleiter 1 bzw. 2 verschiebbar angeordnet ist. Zur Signalübertragung zwischen einem rotierenden und einem nicht rotierenden Teil - beide Teile sind in der Zeichnung nicht dargestellt - sind die Hohlleiter 1, 2 einerseits und die Schlitten 13, 23 andererseits mit je einem dieser beiden Teile gekoppelt. Im folgenden wird angenommen, daß die Schlitten 13, 23 mit dem drehbaren Teil und die Hohlleiter 1, 2 mit dem festen Teil verbunden sind; doch könnte es genauso gut umgekehrt sein. Bei einer Drehung verschieben sich daher die Schlitten 13, 23 auf dem Hohlleiter 1 bzw. 2 und mit ihnen die Antennen 12 bzw. 22 in dem Koppelschlitz 11 bzw. 21.

Obwohl die beiden Hohlleiter 1 und 2 kreisförmig gebogen sind, kann eine elektromagnetische Welle nicht mehr als einmal darin umlaufen, weil sie an jeweils einem Ende durch eine in den Zeichnungen nur schematisch angedeutete Empfangsantenne 14 bzw. 24 abgeschlossen sind, und zwar so, daß die Empfangsantenne 14 nur von im Uhrzeigersinn umlaufenden elektromagnetischen Wellen erreicht wird, während die Empfangsantenne 24 nur von im Gegenuhrzeigersinn umlaufenden elektromagnetischen Wellen erreichbar ist. Zu diesem Zweck sind die die Empfangsantennen tragenden Hohlleiterenden 15 bzw. 25 jeweils in entgegengesetztem Sinn in das Kreisinnere gekrümmt; jedoch wäre es stattdessen auch möglich, die Hohlleiter an einer Stelle reflexionsfrei abzuschließen, wobei die beiden Empfangsantennen auf unterschiedlichen Seiten dieser reflexionsfreien Abschlüsse angeordnet werden müßten.

In jedem der Hohlleiter 1 bzw. 2 ist ein Dämpfungsglied 16 bzw. 26 angeordnet, das den Hohlleiter in zwei Bereiche teilt, die elektromagnetisch voneinander entkoppelt sind, und das den betreffenden Hohlleiter für elektromagnetische Wellen, die aus der Richtung der Empfangsantenne ankommen, reflexionsfrei abschließt. Wenn die Antenne 12 bzw. 22 in den Teil des Koppelschlitzes eintaucht, der über dem Bereich zwischen dem Dämpfungsglied und der Empfangsantenne liegt - dieser Bereich wird im folgenden als "aktiver" Bereich bezeichnet - ist eine Signalübertragung von der Sendeantenne 12 bzw. 22 zur zugeordneten Empfangsantenne 14 bzw. 24 möglich. Taucht die Sendeantenne 12 bzw. 22 hingegen in den anderen - im folgenden als "passiv" bezeichneten - Bereich, ist keine Signalübertragung von der Sendeantenne 12 bzw. 22 zur zugehörigen Empfangsantenne möglich. Bei dem Hohlleiter 1 liegt der aktive Bereich ungefähr in der linken und der passive Bereich ungefähr in der rechten Hälfte des Hohlleiterringes; beim Hohlleiter 2 ist es genau umgekehrt.

Bei der aus der Zeichnung ersichtlichen Stellung des Schlittens 13 bzw. 23 ist nur die Sendeantenne 12 im aktiven Bereich, während die Sendeantenne 22 im passiven Bereich liegt, so daß die von ihr ausgestrahlte Energie die zugehörige Empfangsantenne 24 nicht erreichen kann. Wenn der Schlitten 13 bzw 23 im Uhrzeigersinn auf dem Umfang der Hohlleiterringe 1 bzw. 2 verschoben werden, ändert sich an diesem Zustand, bei dem die von der Sendeantenne 12 ausgestrahlte Energie in Richtung des Pfeiles 17, d.h. im Uhrzeigersinn, die Empfangsantenne 14 erreicht, zunächst nichts; jedoch wird die Laufzeit der elektromagnetischen Welle immer kürzer. Dies dauert an, bis die Schlitten 13 bzw. 23 in die Nähe der Enden 15 bzw. 25 der Hohlleiter 1 bzw. 2 gelangen, so daß der Abstand zwischen Sende- und Empfangsantenne in beiden Fällen gleich ist, wobei beide Sendeantennen im aktiven Bereich sind. Die Laufzeit hat dann ihr Minimum erreicht.

Werden die Schlitten 13 und 23 über den beschriebenen Punkt hinaus im Uhrzeigersinn weitergedreht, gelangt die Sendeantenne 12 in den passiven Bereich des Hohlleiters 1, während die Sendeantenne 22 sich im aktiven Bereich des Hohlleiters bewegt, so daß die elektromagnetischen Wellen sich in Richtung des Pfeiles 27 (Fig. 1), d.h. im Gegenuhrzeigersinn, in dem Hohlleiter 2 fortpflanzen. Dabei nimmt die Laufzeit der elektromagnetischen Wellen mit der weiteren Drehung ständig zu.

Die beiden Dämpfungsglieder 16 und 26 sind in dem den Enden 15 bzw. 25 der Hohlleiter gegenüberliegenden Bereich angeordnet, so daß der aktive Bereich sich jeweils über wenigstens einen halben Umfang des Hohlleiterbogens erstreckt aber auch nicht wesentlich weiter. Erreichen die Schlitten 12 bzw. 22 den Bereich, in dem die Dämpfungsglieder 16 bzw. 26 angeordnet sind, sind beide Sendeantennen (wieder bzw. noch immer) im aktiven Bereich, wobei die Laufzeit der elektromagnetischen Wellen in beiden Hohlleitern gleich und maximal ist. Werden die Schlitten 13 bzw. 23 dann noch weiter im Uhrzeigersinn gedreht, gelangt die Sendeantenne 22 wieder in den passiven Teil des Hohlleiters 2, bis sich wieder die in Fig. 1 dargestellte Lage ergibt.

Gemäß Fig. 3 sind die Sendeantennen 12 und 22 an einen Mikrowellenleistungsteiler angeschlossen, dessen Eingang mit dem Ausgang eines Mikrowellenmischers 4 verbunden sind, der die Signale eines Oszillators 5 mit geeigneter Trägerfrequenz, z. B. 10 GHz, mit denen eines Datensenders 6 mischt, wobei die niedrigste Frequenz in dem in dem Sender 6 emittierten Frequenzgemisch oberhalb einer bestimmten Grenzfrequenz $f_g$ liegt.

Gemäß Fig. 4 werden die von den Antennen 14 bzw. 24 ausgekoppelten Signale in einer Überlagerungsstufe 7 einander überlagert. An den Ausgang der Überlagerungsstufe 7 ist wiederum ein Leistungsteiler 8 angeschlossen, an dessen einen Eingang der eine Eingang einer Mischstufe 10 direkt angeschlossen ist, während an den anderen Ausgang des Leistungsteilers 8 ein schmalbandiges Filter 9 angeschlossen ist, dessen Ausgang mit dem anderen Eingang des Mischers verbunden ist. Die Mittenfrequenz des Filters 9 entspricht der Trägerfrequenz und seine Bandbreite ist kleiner als die doppelte Grenzfrequenz $f_g$, so daß hinter dem Filter 9 lediglich noch das Signal mit der Trägerfrequenz vorhanden ist. Dieses Signal wird in der Mischstufe 9 mit dem empfangenen Signal gemischt, so daß am Ausgang der Mischstufe 9 wiederum ein Signal zur Verfügung steht, das dem von dem Datensender erzeugten Signal entspricht.

## Patentansprüche

1. Vorrichtung für die Mikrowellenübertragung zwischen zwei relativ zueinander drehbaren Teilen mit zwei ringförmigen, konzentrisch angeordneten Hohlleitern, von denen jeder mit einer Empfangs- und einer Sendeantenne versehen ist und auf seinem Umfang einen Koppelschlitz aufweist, wobei die in die beiden Koppelschlitze hineinragenden und darin verschiebbaren Antennen mechanisch miteinander gekoppelt sind und die beiden anderen Antennen fest mit dem zugeordneten Hohlleiter verbunden sind, gekennzeichnet durch folgende Merkmale:
   a) jeder Hohlleiter (1, 2) ist durch die fest mit ihm verbundene Antenne (14, 24) abgeschlossen, die so angeordnet ist, daß sie nur von in einer Richtung umlaufenden elektromagnetischen Wellen erreicht wird, wobei die Umlaufrichtungen (17, 27) in den beiden Hohlleitern einander entgegengesetzt sind,
   b) ein Bereich jedes Hohlleiters ist durch ein Dämpfungsglied (16, 26) von dieser Antenne entkoppelt, so daß der nicht-entkoppelte Bereich annähernd ein halber Kreisbogen ist, und
   c) die beiden Hohlleiter sind so angeordnet, daß mindestens eine der beiden verschiebbaren Antennen in den Bereich des Hohlleiters eintaucht, in dem sich eine Signalübertragung zur festen Antenne (14, 24) ergibt.

2. Vorrichtung nach Anspruch 1, dadurch gekennzeichnet, daß die Sendeantennen (13, 23) in die Koppelschlitze (11, 21) der beiden Hohlleiter (1, 2) eintauchen.

3. Vorrichtung nach Anspruch 1, gekennzeichnet durch ihre Anwendung bei einem Computertomographiegerät.

## Claims

1. An arragement for microwave transmission between two bodies which are rotatable relative to each other having two circular, concentrically arranged waveguides, each of which comprises a receiving and a transmitting aerial and has along its circumference a coupling slot, the aerials which project into the two coupling slots and are movable in the slots being mechanically intercoupled and the two other aerials being fixed to the associated waveguide, characterized in that
   a) each waveguide (1, 2) is closed by the aerial (14, 24) which is fixed to it, and is provided such that they are only reached by electromagnetic waves rotating in one direction of rotation (17, 27) in the two waveguides being opposite to each other,
   b) a sector of each waveguide is decoupled from this aerial by an attenuator (16, 26), so that the non-decoupled sector is substantially half a circle of arc,
   c) the two waveguides are arranged such that at least one of the two movable aerial projects into that sector of the waveguide in which signal transmission to the fixed aerial (14, 24) occurs.

2. An arrangement as claimed in Claim 1, characterized in that the transmitting aerials (13, 23) project into the coupling slots (11, 21) of the two waveguides (1, 2).

3. An arrangement as claimed in Claim 1, characterized in that they are used in a computer tomography apparatus.

## Revendications

1. Dispositif pour la transmission de signaux de micro-ondes entre deux éléments pouvant tourner l'un par rapport à l'autre comportant deux guides d'ondes annulaires concentriques, chaque guide d'ondes étant pourvu d'une antenne réceptrice et d'une antenne émettrice et présentant une fente de couplage sur sa périphérie, étant entendu que les antennes qui s'étendent dans les deux fentes de couplage et qui peuvent y être déplacées sont couplées mécaniquement l'une à l'autre et que les deux autres antennes sont reliées de manière fixe au guide d'ondes associé, caractérisé par les particularités suivantes:
   a) chaque guide d'ondes (1, 2) est terminé par l'antenne (14, 24) qui y est reliée de manière fixe et qui est disposée d'une manière telle qu'elle ne soit atteinte que par des ondes électromagnétiques circulant dans un sens, étant entendu que les sens de circulation (17, 27) dans les deux guides d'ondes sont opposés l'un à l'autre,
   b) une zone de chaque guide d'ondes est désaccouplée par un élément atténuateur (16, 26) de cette antenne, de sorte que la sortie non désaccouplée a à peu près la forme d'un demi-cercle, et
   c) les deux guides d'ondes sont disposés d'une manière telle qu'au moins une des deux antennes déplaçables plonge dans la zone du guide d'ondes dans laquelle une transmission de signaux vers l'antenne fixe (14, 24) se produit.

2. Procédé suivant la revendication 1, caractérisé en ce que les antennes émettrices (13, 23) plongent dans les fentes de couplage (11, 21) des deux guides d'ondes (1, 2).

3.- Dispositif suivant la revendication 1, caractérisé en ce qu'il est utilisé dans un appareil de tomodensitométrie.

FIG.1

FIG.2

FIG.3

FIG.4